(19) Europäisches Patentamt
European Patent Office
Office européen des brevets

(11) Veröffentlichungsnummer: **0 310 903 B1**

(12) **EUROPÄISCHE PATENTSCHRIFT**

(45) Veröffentlichungstag der Patentschrift: **04.12.91**

(51) Int. Cl.$^5$: **C10M 119/30**, C08G 77/38

(21) Anmeldenummer: **88115776.2**

(22) Anmeldetag: **24.09.88**

# (54) Verwendung von Organosilicium-Verbindungen zum Verdicken von Ölen.

(30) Priorität: **09.10.87 DE 3734217**

(43) Veröffentlichungstag der Anmeldung:
**12.04.89 Patentblatt 89/15**

(45) Bekanntmachung des Hinweises auf die Patenterteilung:
**04.12.91 Patentblatt 91/49**

(84) Benannte Vertragsstaaten:
**AT BE DE ES FR GB IT NL**

(56) Entgegenhaltungen:
**EP-A- 0 177 825**

(73) Patentinhaber: **Th. Goldschmidt AG**
**Goldschmidtstrasse 100 Postfach 101461**
**W-4300 Essen 1(DE)**

(72) Erfinder: **Schaefer, Dietmar, Dr.**
**Milchstrasse 40**
**W-4300 Essen 14(DE)**
Erfinder: **Hoffmann, Klaus**
**Ahornstrasse 47**
**W-4300 Essen 1(DE)**

**EP 0 310 903 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von ausgewählten Organopolysiloxanen mit an Siliciumatomen gebundenen langkettigen Alkylgruppen als Verdickungsmittel für Kohlenwasserstofföle, Esteröle und pflanzliche Öle.

Verdickungsmittel für nicht wäßrige Systeme, insbesondere Verdickungsmittel für Mineralöle, Esteröle und pflanzliche Öle werden insbesondere in der Kosmetik und zur Herstellung von Schmiermitteln verwendet. Je nach Verwendungszweck werden unterschiedliche Verdickungsmittel eingesetzt.

Als Verdickungsmittel werden in der Kosmetik höhere Fettalkohole, Fettsäuren und deren Ethoxylierungsprodukte, Glycerinmonofettsäureester, pflanzliche und andere Wachse, polymere Verbindungen und Metallseifen verwendet. Diese in kosmetischen Zubereitungen verwendeten Verdickungsmittel müssen hautverträglich sein und dürfen bei der bestimmungsgemäßen Verwendung der Kosmetika keine störenden Effekte verursachen.

Zur Verdickung von Schmiermitteln werden insbesondere Alkali-, Erdalkali-, Zink- und Aluminiumstearate verwendet. Es haben sich auch aromatische Polyharnstoffe als brauchbar erwiesen. Man verwendet zur Verdickung ferner hochdisperse Kieselsäure, Ruß und aufbereitete Tonmineralien, wie Bentonite.

Weitere Verwendungsmöglichkeiten für Verdickungsmittel ergeben sich zur Beeinflussung und/oder Stabilisierung der Viskosität von Treibstoffen und zur Herstellung von Brandsätzen für militärische Einsatzzwecke.

Der vorliegenden Erfindung liegt die Aufgabe zugrunde, neue Verdikkungsmittel zu finden, die vielseitig verwendbar, in möglichst geringen Konzentrationen wirksam und physiologisch unbedenklich sind, um ihre Verwendbarkeit auch im kosmetischen und pharmazeutischen Bereich zu ermöglichen.

Überraschenderweise wurde gefunden, daß ausgewählte modifizierte Organopolysiloxane verdickende Eigenschaften aufweisen und dabei Effekte zeigen, die ihre Verwendung in der Kosmetik und Pharmazie, aber auch in bestimmten Bereichen der Technik besonders empfehlen.

Gegenstand vorliegender Erfindung ist deshalb die Verwendung von Verbindungen der allgemeinen Formel

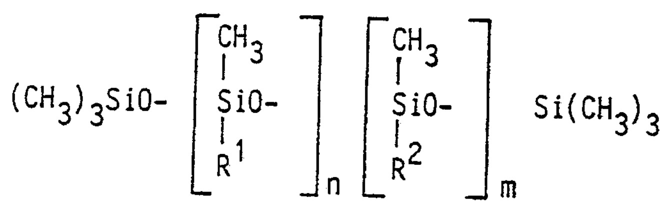

I

wobei

$R^1$ ein Alkylrest mit 18 bis 36 Kohlenstoffatomen ist,

$R^2$ ein Wasserstoffrest, ein Methylrest oder ein Rest der allgemeinen Formel $-(CH_2)_xR^3$ ist,
wobei $R^3$ ein $COOR^4$-Rest, in dem $R^4$ ein Wasserstoffrest oder ein Alkylrest mit 1 bis 4 Kohlenstoffatomen ist, oder
ein $-O[C_rH_{2r}O-]_yR^5$-Rest, in dem $R^5$ ein Wasserstoffrest, ein Alkylrest mit 1 bis 4 Kohlenstoffatomen oder ein Acylrest,
$r = 2$ oder 3 und $y \geqq 0$ ist,
$x = 2$ bis 12 ist,
$n \geqq 20$ und $m = 0$ bis $0,5\ n$ ist,

als Verdickungsmittel für Kohlenwasserstofföle, Esteröle und pflanzliche Öle in Mengen von 2 bis 10 Gew.-%, bezogen auf die Öle.

$R^1$ ist ein Alkylrest mit 18 bis 36 Kohlenstoffatomen, wobei Alkylreste mit 22 bis 36 Kohlenstoffatomen bevorzugt sind.

$R^2$ ist ein Wasserstoffrest, ein Methylrest oder ein Rest der allgemeinen Formel $-(CH_2)_xR^3$. x ist dabei eine ganze Zahl von 2 bis 12. $R^3$ hat entweder die Bedeutung eines $-COOR^4$-Restes oder die Bedeutung des Restes $-O[C_rH_{2r}O-]_yR^5$. $R^4$ ist ein Wasserstoffrest oder ein Alkylrest mit 1 bis 4 Kohlenstoffatomen; $R^5$ ist ein Wasserstoffrest, ein Alkylrest mit 1 bis 4 Kohlenstoffatomen oder ein Acylrest, insbesondere ein Acetylrest. r hat einen Wert von 2 oder 3, wobei im durchschnittlichen Molekül beide Werte nebeneinander vorliegen können und deshalb auch gebrochene Werte zwischen 2 und 3 möglich sind. y ist $\geqq 0$.

Die Anzahl der difunktionellen Siloxyeinheiten n ist $\geqq 20$; m hat einen Wert von 0 bis $0,5\ n$.

Zur weiteren Erläuterung des Aufbaus der erfindungsgemäß zu verwendenden modifizierten Organopolysiloxane dienen die folgenden Strukturformeln typischer und bevorzugter Verbindungen.

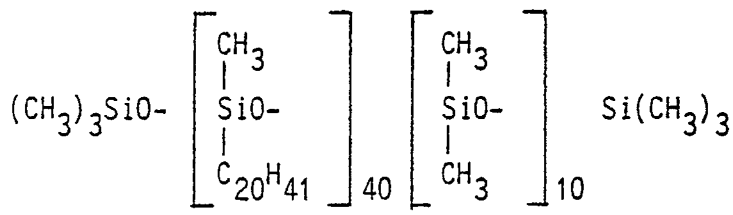

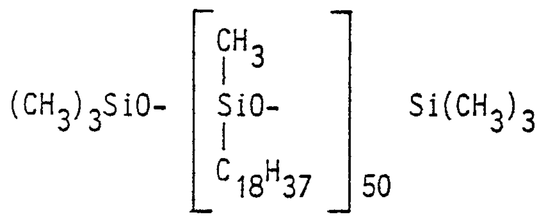

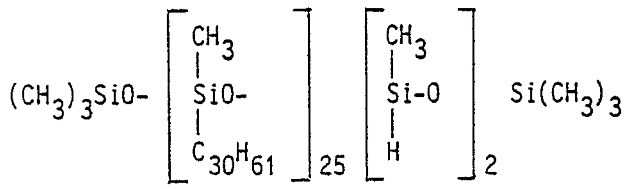

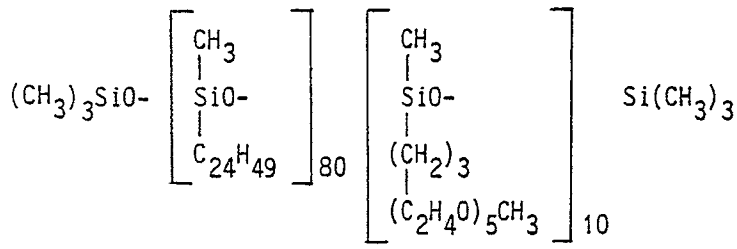

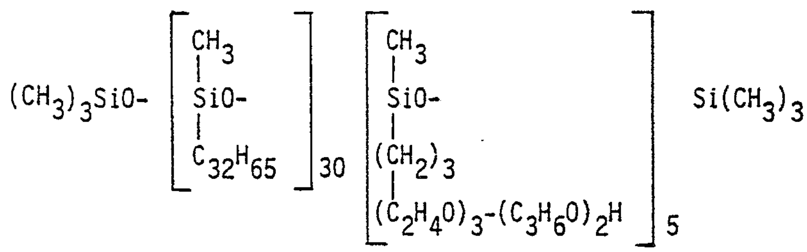

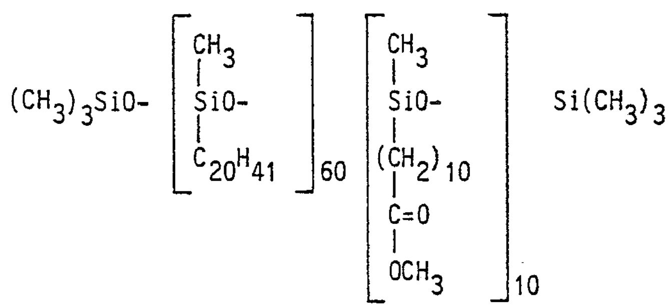

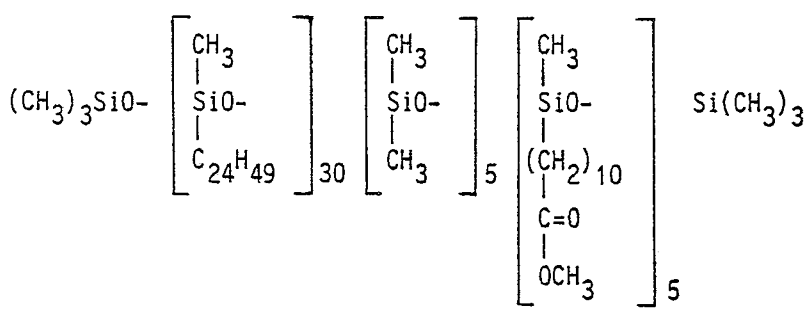

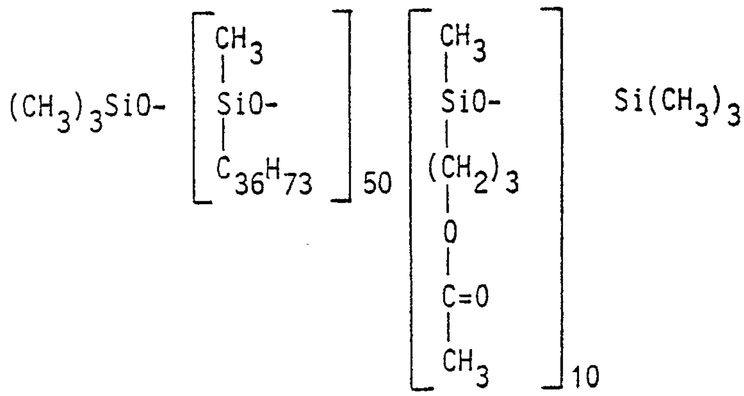

Die erfindungsgemäß zu verwendenden Verbindungen können in an sich bekannter Weise aus Wasserstoff-methyl-polysiloxanen der allgemeinen Formel

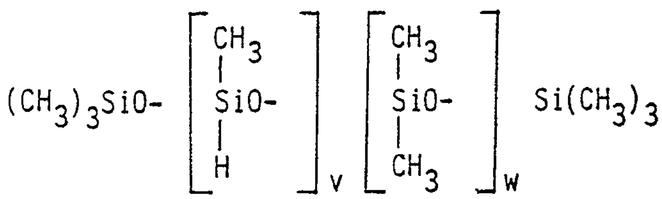

II

wobei v + w = n + m ist, durch hydrosilylierende Anlagerung von Alkenen mit 18 bis 36 Kohlenstoffatomen und endständiger olefinischer Doppelbindung, und gegebenenfalls Verbindungen der allgemeinen Formel

$CH_2 = CH\text{-}(CH_2)_{x\text{-}2}R^3$

in Gegenwart von Katalysatoren, insbesondere Platinverbindungen, hergestellt werden.

Die modifizierten Organopolysiloxane der allgemeinen Formel I sind wachsartige Verbindungen. Sie lösen sich in den Ölen bei Temperaturen oberhalb 40° C. Beim Abkühlen auf Raumtemperatur bilden die erfindungsgemäß zu verwendenden modifizierten Polysiloxane in den Ölen vermutlich eine gerüstartige kristalline Struktur aus, die Ursache für die Strukturviskosität der erfindungsgemäß verdickten Öle sein kann. Läßt man auf die verdickten Öle Scherkräfte, z.B. mittels eines scherkraftreichen Rührers, einwirken, verflüssigen sich die verdickten Zubereitungen. Mit erneuter Ausbildung des Strukturgerüstes im Öl steigt dessen Viskosität wieder an. Der gleiche Effekt ist zu beobachten, wenn die erfindungsgemäß verdickte Zubereitung auf eine Temperatur oberhalb 40° C erwärmt wird. Oberhalb dieses Temperaturbereiches löst sich das modifizierte Organopolysiloxan im Öl und die Zubereitung weist im wesentlichen die Viskosität des Grundöles auf. Wird die Zubereitung auf eine Temperatur unterhalb des vorgenannten Temperturbereiches abgekühlt, baut sich im Öl wieder die die Verdickung bewirkende Struktur aus.

Dieses Verhalten kann insbesondere bei kosmetischen oder pharmazeutischen Zubereitungen ausgenutzt werden. So lassen such beispielsweise Zubereitungen herstellen, die bei Raumtemperatur hohe Viskosität aufweisen, sich aber beim Auftragen auf die Haut verflüssigen und dadurch leicht appliziert, auf der Haut verteilt und gegebenenfalls in diese eingerieben werden können.

Die mit den modifizierten Organopolysiloxanen erfindungsgemäß verdickten Öle lassen sich leicht mit einem Spatel rühren. Sie sind weich und geschmeidig. Sie sind weder zäh noch klebrig.

Als Beispiele für die erfindungsgemäß zu verdickenden Kohlenwasserstofföle können Paraffinöle und Testbenzin genannt werden.

Beispiele für Esteröle sind Isooctylstearat und Isooctadecylisononanat.

Beispiele für pflanzliche Öle sind Sonnenblumenöl und Sojaöl.

Die erfindungsgemäß zu verwendenden Verdickungsmittel werden in Mengen von 2 bis 10 Gew.-%, vorzugsweise 5 bis 10 Gew-%, bezogen auf Öl, eingesetzt.

In den folgenden Beispielen werden die Eigenschaften der erfindungsgemäß zu verwendenden modifizierten Polysiloxane und das Strukturverhalten der verdickten Öle näher erläutert.

Die Viskositäten wurden mit einem Rotationsviskosimeter (Rheomat 115 der Firma Contraves AG, Zürich) nach DIN 125 (Modul 7/7) bestimmt.

In den Beispielen wird die Viskosität als Funktion des Geschwindigkeitsgefälles angegeben. Die Viskosität wird bei jeder Geschwindigkeitsstufe nach 15 Sekunden bestimmt. Zwischen jeder Geschwindigkeitsstufe werden die verdickten Öle 15 Sekunden mechanisch nicht beansprucht.

Bei der graphischen Darstellung der Viskosität (mPas) als Funktion des Geschwindigkeitsgefälles (s-1) ist zu beachten, daß die einzelnen Geschwindigkeitsstufen in Pfeilrichtung durchlaufen werden.

Beispiel 1

5 g einer Verbindung der allgemeinen Formel

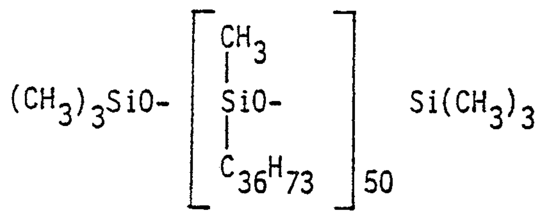

werden in 95 g Paraffinöl einer Ausgangsviskosität von ca. 150 mPas bei 80 °C gelöst und ohne Rühren langsam abgekühlt.

Die Viskosität des verdickten Paraffinöles bei 25 °C als Funktion des Geschwindigkeitsgefälles ist in Fig. 1 dargestellt.

Beispiel 2

Das Beispiel 1 wird wiederholt, jedoch wird anstelle der 5 %igen Lösung eine 8 %ige Lösung untersucht (Fig. 2).

Beispiel 3

In diesem Beispiel wird der Einfluß der Temperatur auf die Viskosität des erfindungsgemäß verdickten Paraffinöles bei einem konstanten Geschwindigkeitsgefälle von 6,65 s-1 untersucht (Fig. 3).

Beispiel 4

5 g einer Verbindung der allgemeinen Formel

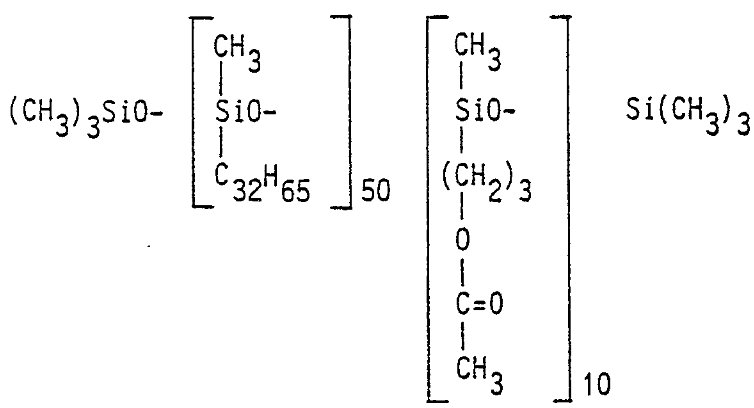

werden in 95 g Isooctylstearat bei 80 °C gelöst und ohne Rühren langsam abgekühlt.

Die Viskosität des verdickten Isooctylstearates als Funktion des Geschwindigkeitsgefälles bei 25 °C ist

in Fig. 4 dargestellt.

Beispiel 5

5 g einer Verbindung der allgemeinen Formel

$$(CH_3)_3SiO-\left[\begin{array}{l} CH_3 \\ | \\ SiO- \\ | \\ C_{36}H_{73} \end{array}\right]_{50} \left[\begin{array}{l} CH_3 \\ | \\ SiO- \\ | \\ (CH_2)_3 \\ | \\ (C_2H_4O)_6CH_3 \end{array}\right]_{10} Si(CH_3)_3$$

werden in 95 g Isooctylstearat bei 80 ° C gelöst und ohne Rühren langsam abgekühlt.

Die Viskosität des verdickten Isooctylstearates als Funktion des Geschwindigkeitsgefälles bei 25 ° C ist in Fig. 5 dargestellt.

Beispiel 6

5 g einer Verbindung der allgemeinen Formel

$$(CH_3)_3SiO-\left[\begin{array}{l} CH_3 \\ | \\ SiO- \\ | \\ C_{26}H_{53} \end{array}\right]_{50} Si(CH_3)_3$$

werden in 95 g Paraffinöl einer Viskosität von ca. 150 mPas bei 80 ° C gelöst und ohne Rühren langsam abgekühlt.

Die Viskosität des verdickten Paraffinöles bei 25 ° C als Funktion des Geschwindigkeitsgefälles ist in Fig 6 dargestellt.

Beispiel 7

Beispiel 1 wird wiederholt, jedoch wird das Paraffinöl durch Testbenzin eines Siedebereiches von 145 bis 200 ° C ersetzt.

Die Viskosität des verdickten Testbenzins bei 25 ° C als Funktion des Geschwindigkeitsgefälles ist in Fig. 7 dargestellt.

Beispiel 8

Beispiel 6 wird wiederholt, jedoch wird das Paraffinöl durch Testbenzin eines Siedebereiches von 145 bis 200 ° C ersetzt.

Die Viskosität des verdickten Testbenzins bei 25 ° C als Funktion des Geschwindigkeitsgefälles ist in Fig. 8 dargestellt.

**Patentansprüche**

**1.** Verwendung von Verbindungen der allgemeinen Formel

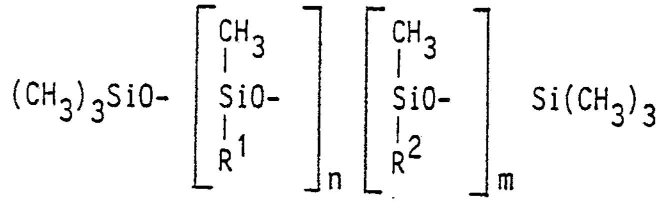

wobei
$R^1$ ein Alkylrest mit 18 bis 36 Kohlenstoffatomen ist,
$R^2$ ein Wasserstoffrest, ein Methylrest oder ein Rest der allgemeinen Formel $-(CH_2)_xR^3$ ist, wobei $R^3$ ein $COOR^4$-Rest, in dem $R^4$ ein Wasserstoffrest oder ein Alkylrest mit 1 bis 4 Kohlenstoffatomen ist, oder ein $-O[C_rH_{2r}O-]_yR^5$-Rest, in dem $R^5$ ein Wasserstoffrest, ein Alkylrest mit 1 bis 4 Kohlenstoffatomen oder ein Acylrest,
$r = 2$ oder 3 und $y \geqq 0$ ist,
$x = 2$ bis 12 ist,
$n \geqq 20$ und $m = 0$ bis 0,5 n ist,
als Verdickungsmittel für Kohlenwasserstofföle, Esteröle und pflanzliche Öle in Mengen von 2 bis 10 Gew.-%, bezogen auf die Öle.

## Claims

1. Use of compounds of the general formula

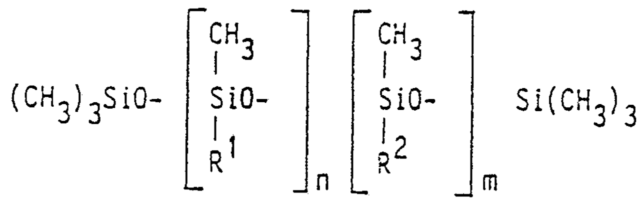

where
$R^1$ is an alkyl radical having 18 to 36 carbon atoms,
$R^2$ is a hydrogen atom, a methyl radical or a radical of the general formula $-(CH_2)_xR^3$, where $R^3$ is a $COOR^4$ radical in which $R^4$ is a hydrogen atom or an alkyl radical having 1 to 4 carbon atoms, or is a $-O[C_rH_{2r}O-]_yR^5$ radical in which $R^5$ is a hydrogen atom, an alkyl radical having 1 to 4 carbon atoms or an acyl radical,
$r = 2$ or 3 and $y \geqq 0$, and
$x = 2$ to 12,
$n \geqq 20$ and $m = 0$ to 0.5 n,
as thickeners for hydrocarbon oils, ester oils and vegetable oils in amounts from 2 to 10% by weight based on the oils.

## Revendications

1. Utilisation de composés de formule générale :

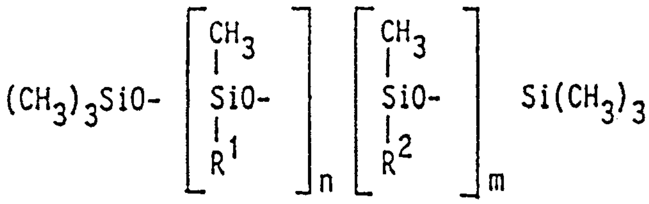

dans laquelle

$R^1$ représente un groupe alcoyle comportant 18 à 36 atomes de carbone,

$R^2$ représente un atome d'hydrogène, un groupe méthyle ou un groupe répondant à la formule générale $-(CH_2)_xR^3$,

$R^3$ étant un groupe $COOR^4$, dans lequel $R^4$ représente un atome d'hydrogène ou un groupe alcoyle comportant 1 à 4 atomes de carbone, ou

un groupe $-O[C_rH_{2r}O-]_yR^5$, dans lequel $R^5$ représente un atome d'hydrogène, un groupe alcoyle comportant 1 à 4 atomes de carbone, ou un groupe acyle,

$r = 2$ ou 3 et $y \geqq 0$,

$x = 2$ à 12,

$n \geqq 20$ et $m = 0$ à $0,5\ n$,

comme agents épaississants d'huiles hydrocarburées, d'huiles-esters et d'huiles végétales, à raison de 2 à 10 96 en poids par rapport aux huiles.

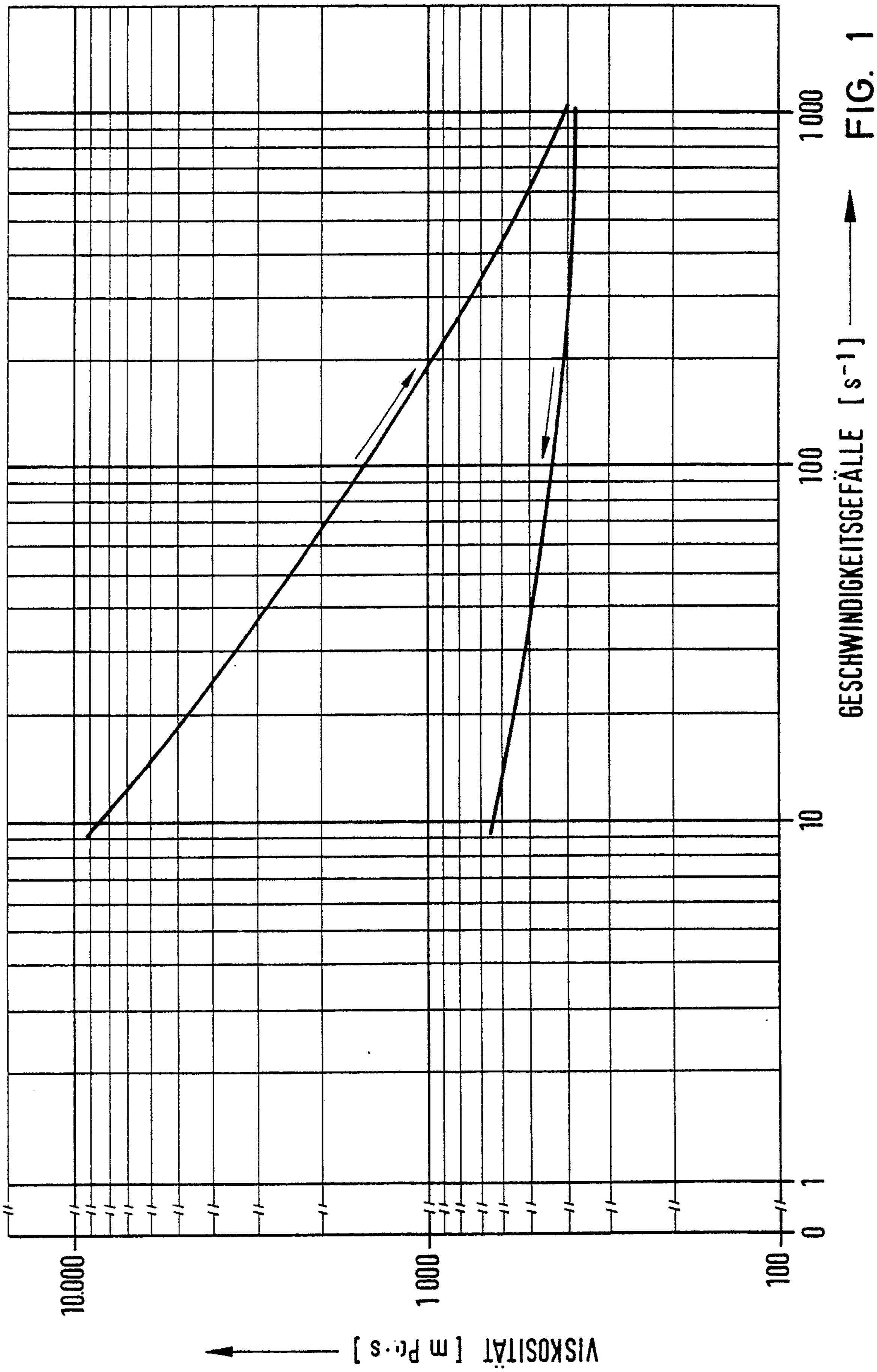
10.000
1000
100
0
1
10
100
1000
VISKOSITÄT [m Pa·s]
GESCHWINDIGKEITSGEFÄLLE [s⁻¹]

FIG. 1

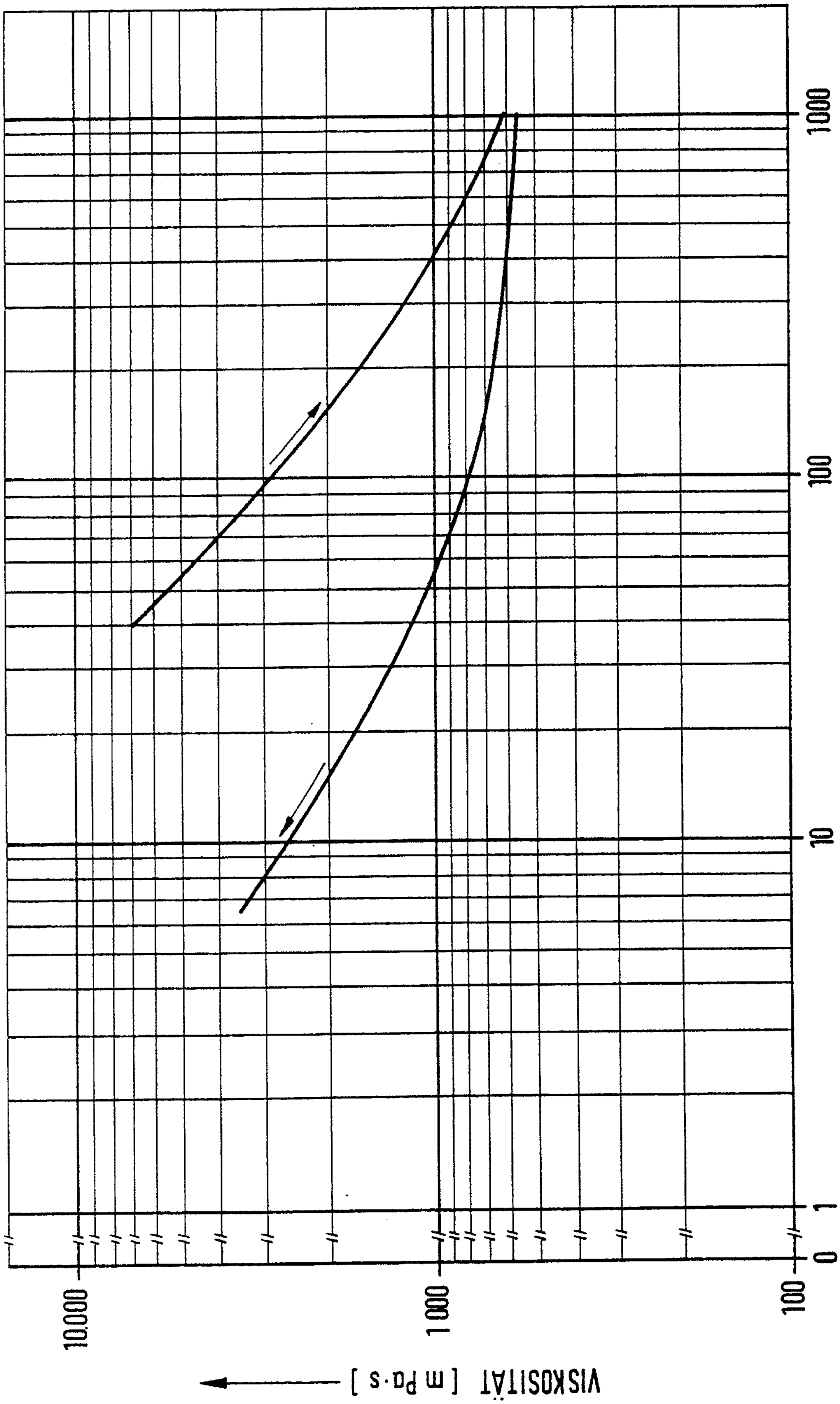

10.000
1 000
100
0
1
10
100
1000
VISKOSITÄT [ m Pa·s ]
GESCHWINDIGKEITSGEFÄLLE [ s-1 ]


FIG. 2

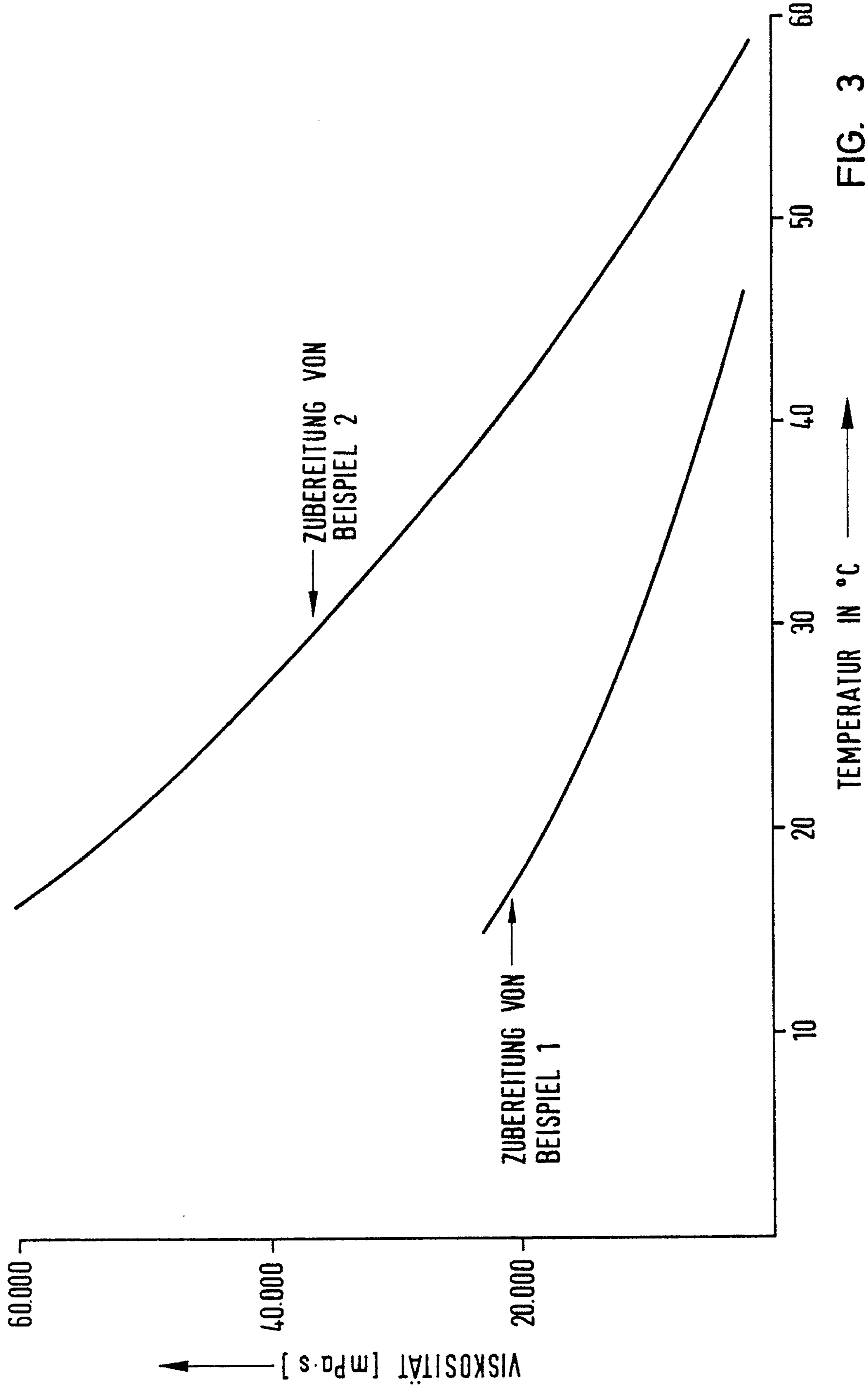
ZUBEREITUNG VON
BEISPIEL 2
ZUBEREITUNG VON
BEISPIEL 1
60.000
40.000
20.000
VISKOSITÄT [mPa·s]
10
20
30
40
50
60
TEMPERATUR IN °C

FIG. 3

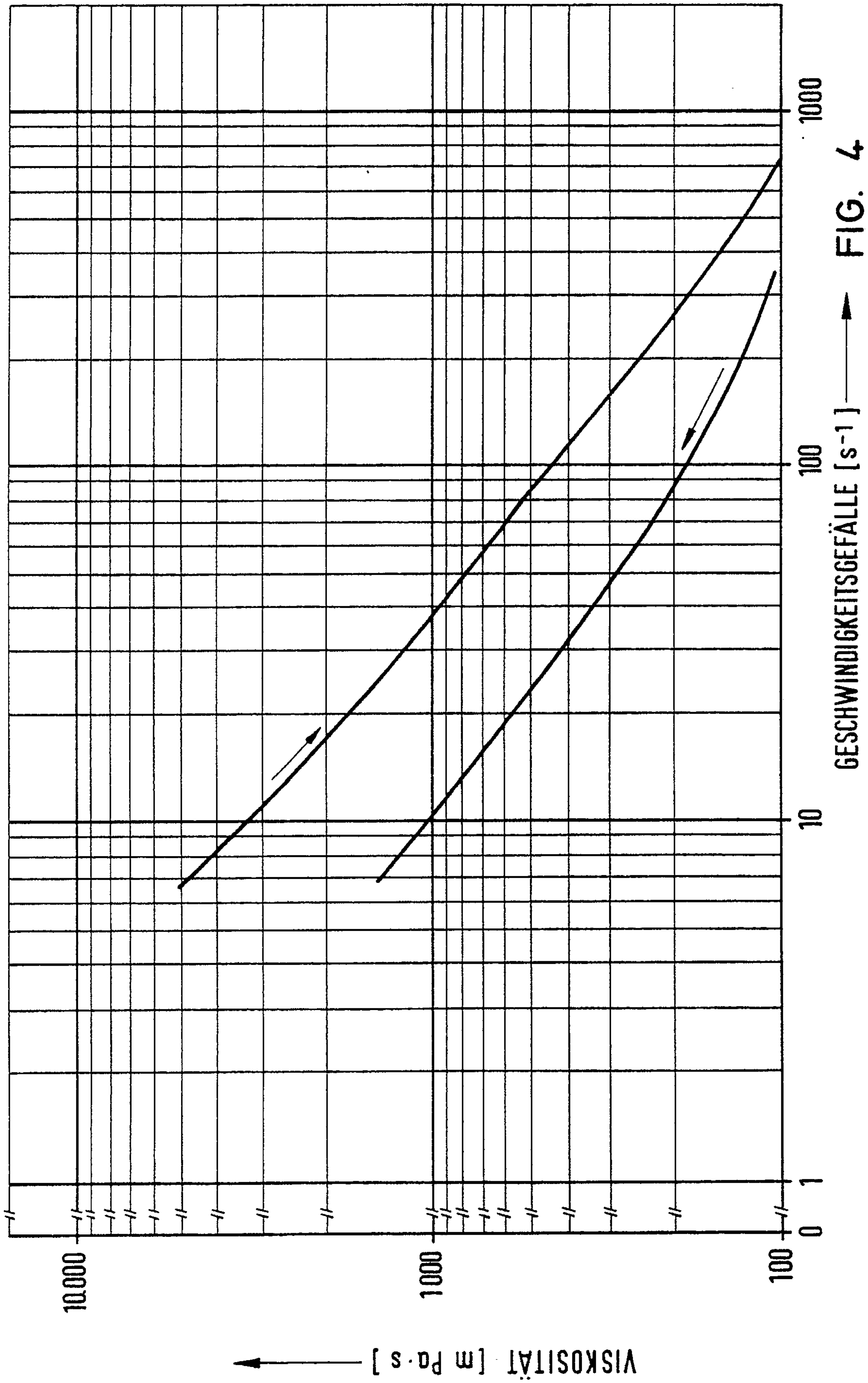
1000
100
10
1
0
GESCHWINDIGKEITSGEFÄLLE [s-1]
10.000
1000
100
VISKOSITÄT [m Pa·s]

FIG. 4

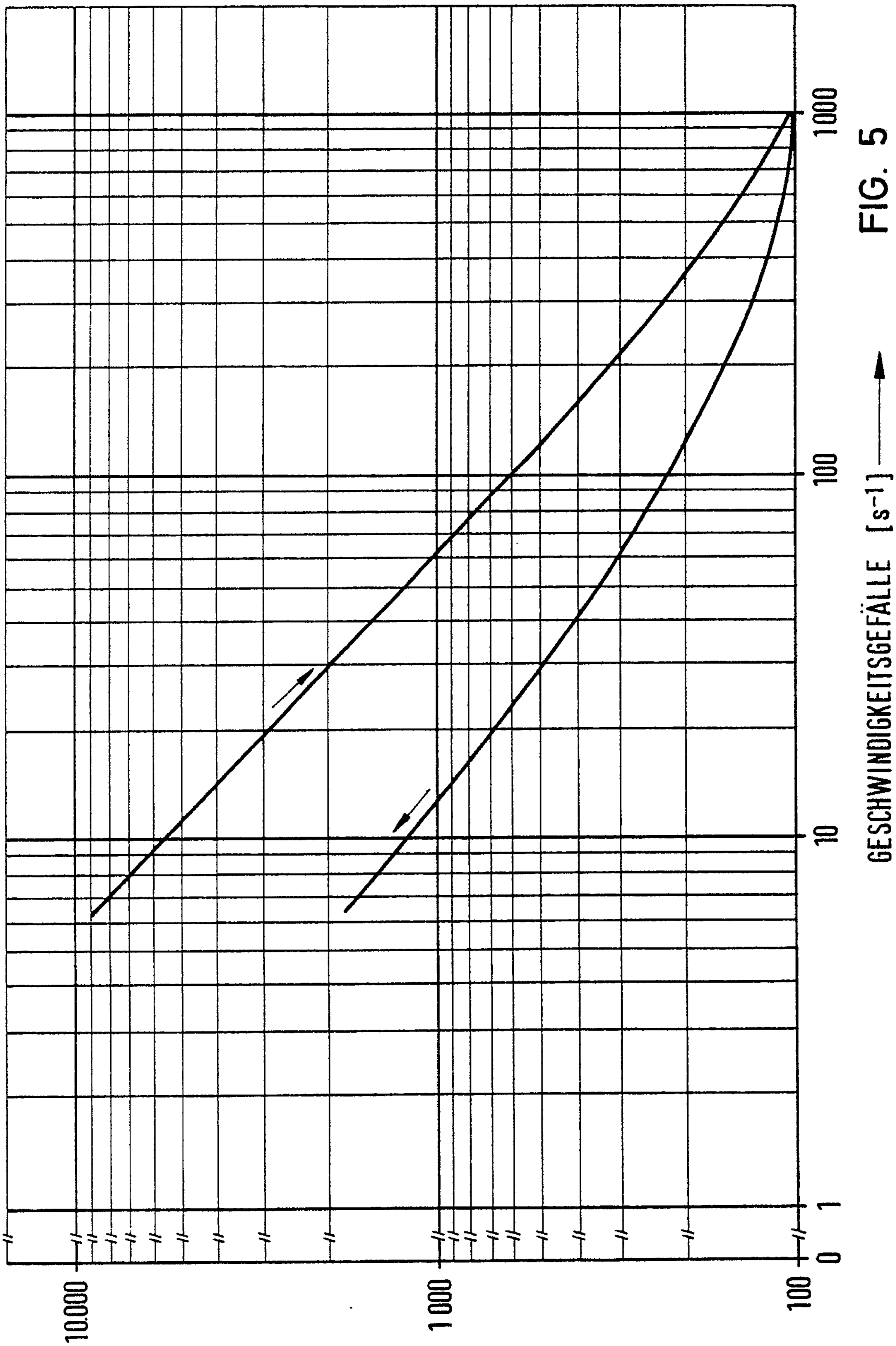


FIG. 5

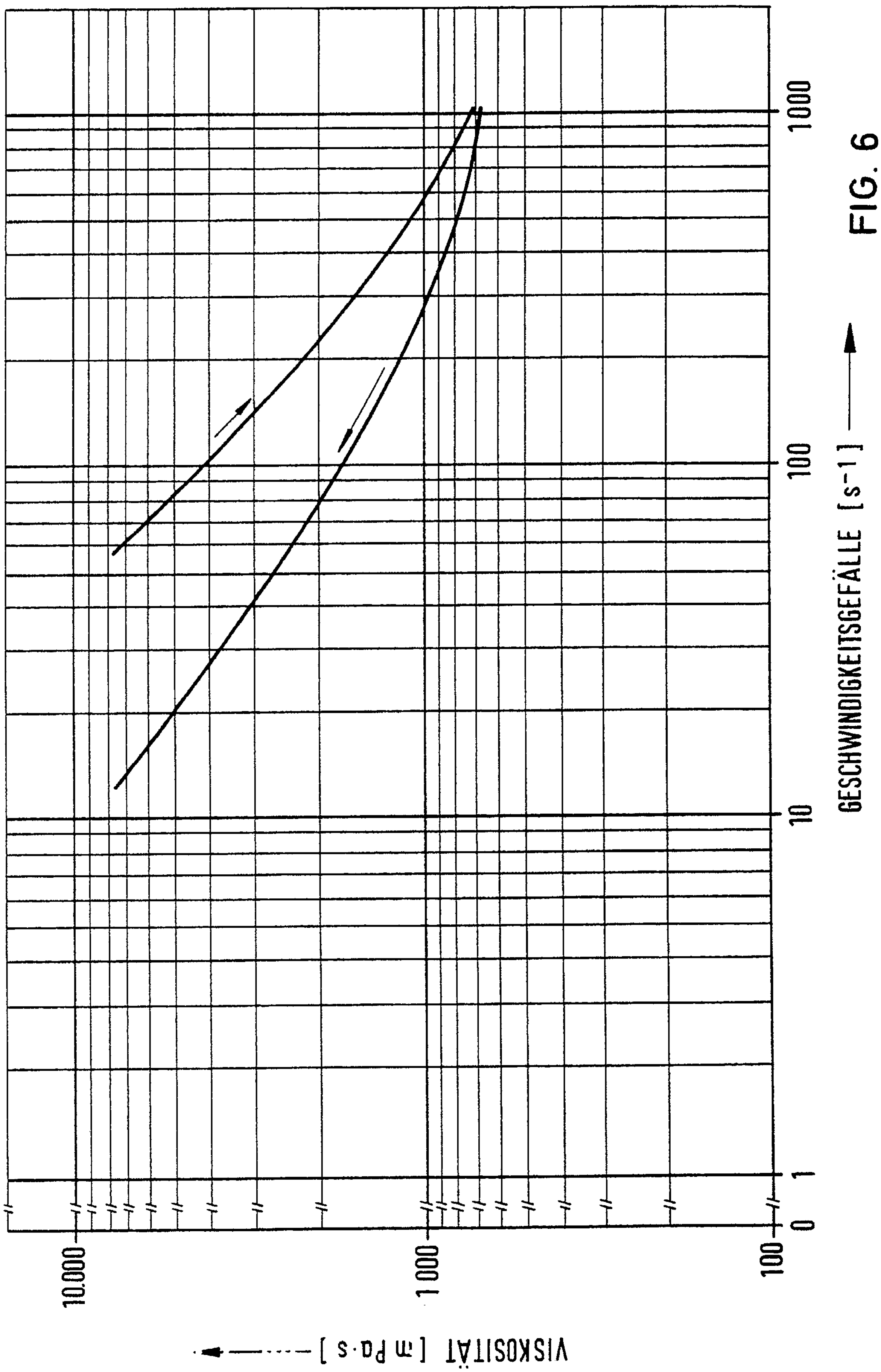
1000
100
10
1
0
GESCHWINDIGKEITSGEFÄLLE [s⁻¹]
10.000
1 000
100
VISKOSITÄT [m Pa·s]

FIG. 6

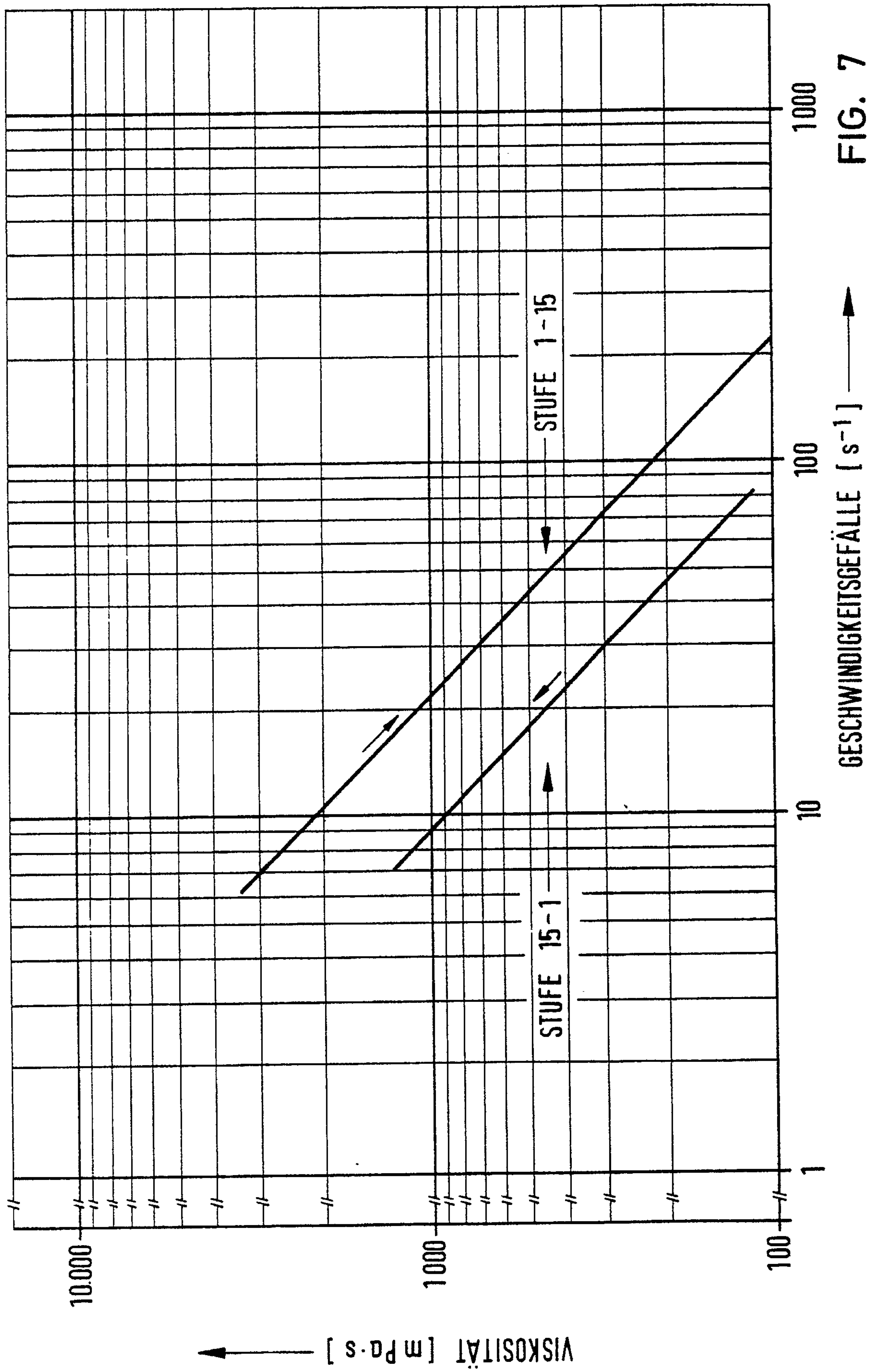
STUFE 1-15
STUFE 15-1
GESCHWINDIGKEITSGEFÄLLE [ $s^{-1}$ ]
1
10
100
1000
VISKOSITÄT [m Pa·s]
10.000
1000
100

FIG. 7

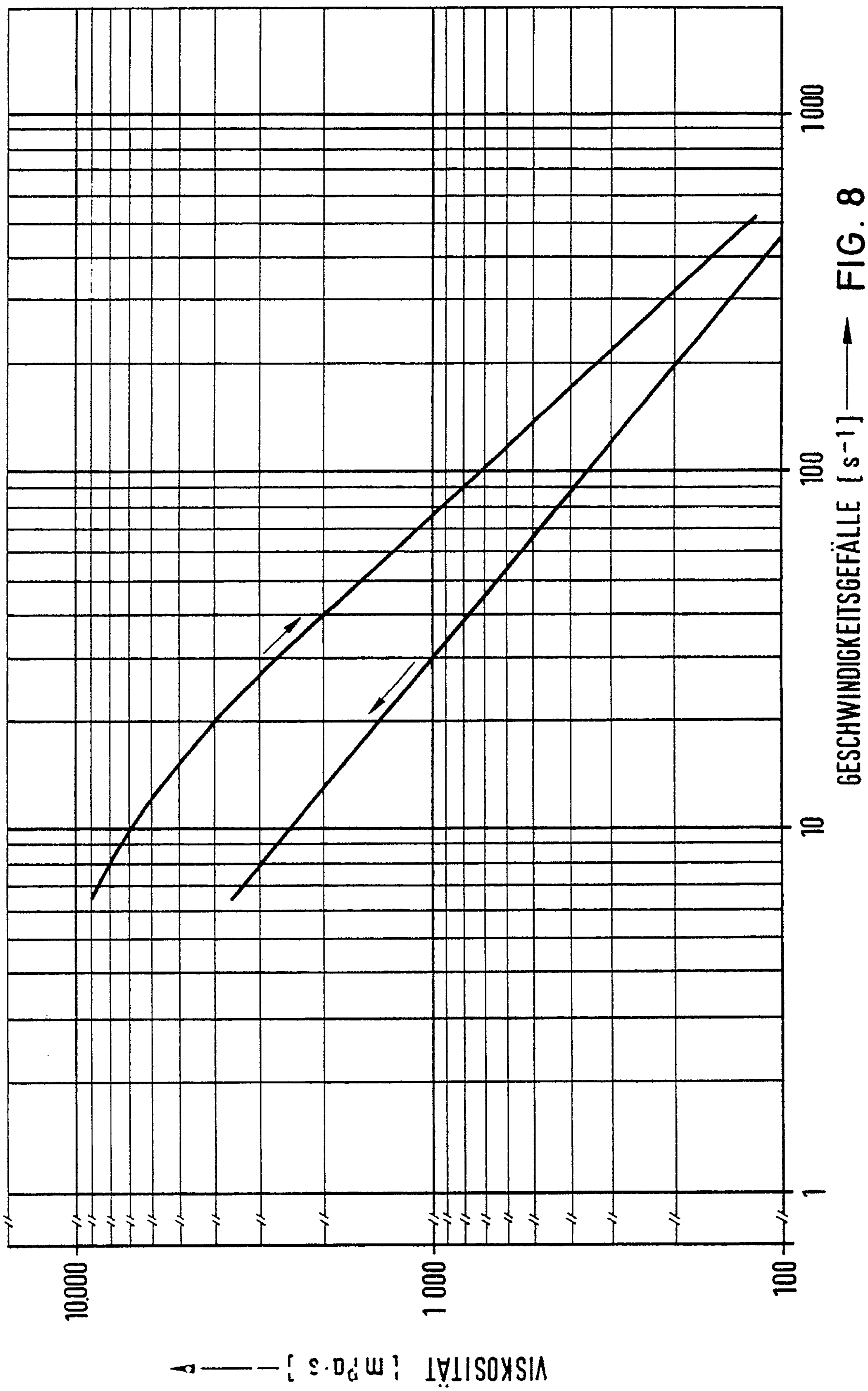

10.000
1 000
100
VISKOSITÄT [mPa·s]
1
10
100
1000
GESCHWINDIGKEITSGEFÄLLE [s-1]


FIG. 8